# FASCICULE DE BREVET EUROPEEN

(11) **EP 2 350 640 B1**
(45) Date de publication et mention de la délivrance du brevet: **10.08.2016**
(21) Numéro de dépôt: 09760178.5
(22) Date de dépôt: 20.10.2009
(51) Int. Cl.: G01N 33/28, G01N 33/30

(54) **DISPOSITIF POUR LE CONTRÔLE DE LA QUALITÉ D'UN LUBRIFIANT ET PROCÉDÉ POUR LE CONTRÔLE DU FONCTIONNEMENT D'UN ÉQUIPEMENT INDUSTRIEL UTILISANT UN LUBRIFIANT**
VORRICHTUNG ZUR SCHMIERMITTELQUALITÄTSKONTROLLE UND VERFAHREN ZUR STEUERUNG DES BETRIEBS EINER GROSSTECHNISCHEN VORRICHTUNG UNTER VERWENDUNG EINES SCHMIERMITTELS
DEVICE FOR CONTROLLING LUBRICANT QUALITY AND METHOD FOR CONTROLLING THE OPERATION OF AN INDUSTRIAL APPARATUS USING A LUBRICANT

(30) Priorité: 22.10.2008 FR 0805846
(43) Date de publication de la demande: 03.08.2011
(73) Titulaire: TOTAL MARKETING SERVICES, 92800 Puteaux (FR)
(72) Inventeur: DOYEN, Valérie, F-38080 Four (FR); HAIHAL, BENMEDAKHENE, Imane, F-60200 Compiegne (FR); DE WIT, Jean-Claude, F-92500 Rueil Malmaison (FR)
(74) Mandataire: Largeau, Béatrice
(86) Numéro de dépôt international: PCT/FR2009/051999
(87) Numéro de publication internationale: WO 2010/046591

(56) Documents cités:
- WO-A-03/091550
- WO-A-2007/110504
- US-A1- 2007 245 811

## Description

La présente invention concerne le domaine de la mesure, en continu ou en discontinu, de l'évolution de la qualité d'un lubrifiant en circulation dans un équipement industriel.

L'invention concerne plus précisément un dispositif permettant de mesurer l'évolution des caractéristiques d'un lubrifiant au sein d'un équipement industriel, tel un matériel en fonctionnement, par exemple un moteur ou une machine.

La présente invention concerne également un procédé pour le contrôle du fonctionnement d'un équipement industriel, par exemple un moteur, notamment thermique, ou une machine, par exemple tournante, grâce au suivi de la qualité de son lubrifiant.

Plus précisément la présente invention concerne le contrôle des moteurs marins, par exemple de type quatre-temps ou deux-temps, par l'analyse des huiles en circulation et/ou récupérées dans lesdits moteurs.

Ces applications seront plus particulièrement décrites dans la suite de la présente description, mais bien entendu, le dispositif et le procédé objets de la présente invention peuvent s'appliquer à tout type de contrôle d'un équipement industriel comprenant la circulation, ou la présence, d'un lubrifiant pour son fonctionnement.

En effet, un lubrifiant est généralement utilisé dans bon nombre d'équipements industriels, en fait dans pratiquement tous les moteurs et machines. Ledit lubrifiant peut notamment contribuer à de nombreuses fonctions essentielles, comme :
- la réduction de la consommation d'énergie,
- la limitation des émissions atmosphériques,
- la limitation de l'usure,
- le refroidissement, notamment des parties dites sensibles,
- la protection contre la corrosion, et/ou
- l'amélioration de l'étanchéité.

Les machines peuvent être des groupes électrogènes, des turbines, notamment à gaz ou à vapeur, des réducteurs, des multiplicateurs, des presses hydrauliques, des machines outils, des compresseurs, des transformateurs, voire des équipements thermiques utilisant des circuits d'huile chaude.

Plus précisément, les moteurs peuvent être des équipements industriels installés dans tout système mobile du domaine dit « automotive », par exemple dans des navires, des camions, des véhicules, des voitures de compétition, des matériels agricoles, des engins de manutention et/ou de transports, des cars ou des trains.

Dans le domaine des moteurs pour navires, on distingue généralement les moteurs marins fonctionnant à quatre-temps et deux-temps. Les moteurs 4-temps peuvent avoir un cycle de fonctionnement rapide ou semi rapide. Les moteurs du premier type sont des moteurs de gamme de puissance faible à modérée (15 à 200 kW par cylindre) dérivés des moteurs terrestres et utilisant des carburants de type distillats tels que le diesel marin à faible teneur en soufre. Leur vitesse de fonctionnement est en général de l'ordre de 1200 tours par minute. Ces moteurs sont utilisés pour la propulsion de navires de faible tonnage et comme unité de génération d'électricité à bord de navires plus importants.

Les moteurs marins quatre-temps semi rapides sont des moteurs de gamme de puissance moyenne à élevée (500 à 2000 kW par cylindre) de conception proche de celle des moteurs quatre-temps rapides mais qui diffèrent de ces derniers par la plus grande taille de l'ensemble piston cylindre. Ces moteurs utilisent en général un carburant appelé fioul soute ou fioul lourd (*Heavy Fuel Oil*) qui, du fait de sa forte teneur en soufre, requiert un lubrifiant à indice total de base (BN comme Base Number en anglais) élevé, généralement compris entre 30 et 65 mg de KOH/g de lubrifiant.

La vitesse de fonctionnement des moteurs marins quatre-temps semi rapides est comprise entre 300 et 600 tours par minute. Ces moteurs sont utilisés pour la propulsion de nombreux navires, tels les unités de type Ro-Ro, les cargos, les tankers, les ferries, voire même certains porte containers. Ils peuvent en outre être utilisés comme unités de génération d'électricité à bord de navires de grande taille ou dans des centrales diesel électriques.

Les moteurs marins quatre-temps ont un fonctionnement très différent de celui des moteurs marins deux-temps, en particulier en ce qui concerne leur mode de lubrification. En effet, les moteurs marins deux-temps sont des moteurs très lents, de gamme de puissance élevée à très élevée (2000 à 6000 kW par cylindre). Ces moteurs sont toujours constitués de deux parties lubrifiées séparément, à savoir l'ensemble piston cylindre lubrifié à graissage perdu par l'huile cylindre très visqueuse, généralement de grade SAE 50 ou 60, et l'arbre manivelle lubrifié par l'huile système peu visqueuse, généralement de grade SAE 30.

Ces moteurs deux-temps utilisent en général un carburant appelé fioul soute qui, de part sa teneur très élevée en soufre, requiert généralement des huiles cylindres à nombre total de base (BN) pouvant aller jusqu'à 100 mg de KOH/g d'huile.

En circulant à travers les circuits internes des moteurs ou des machines le lubrifiant va recueillir des contaminations et/ou des matériaux d'usures desdits moteurs ou machines. De ce fait, le lubrifiant va se dégrader plus ou moins rapidement au cours du fonctionnement de l'équipement industriel. Le lubrifiant contient donc de nombreuses informations précieuses sur le fonctionnement du moteur ou de la machine, sur les éventuelles contaminations, sur son état mécanique et par conséquent sur ses performances fonctionnelles présentes et à venir.

Les différentes dégradations et/ou contaminations du lubrifiant en cours de service dans le moteur ou la machine peuvent conduire à des corrosions et/ou des dégradations irréversibles des parties sensibles du moteur ou de la machine, conduisant par exemple à la modification du BN du lubrifiant, la modification de sa viscosité, l'apparition de particules métalliques, l'évolution de la teneur en eau du lubrifiant, etc.

Ce sont ces paramètres que la présente invention permet de mesurer et d'analyser, en continu ou en discontinu, pour obtenir un meilleur contrôle, une meilleure utilisation et une meilleure protection du moteur ou de la machine.

Dans le cas des moteurs (et plus précisément d'un moteur marin deux-temps lent à crosse) les résidus de combustion contenant des gaz acides sont en contact direct avec l'huile lubrifiante au sein de l'ensemble piston cylindre.

Ces gaz acides peuvent se former à partir de la combustion des fiouls, ce sont notamment des oxydes de soufre (SO₂, SO₃) qui sont ensuite hydrolysés lors du contact avec l'humidité présente dans les gaz de combustion et/ou dans l'huile. Cette hydrolyse génère de l'acide sulfureux (HSO₃) ou sulfurique (H₂SO₄).

Les acides formés doivent être neutralisés, notamment pour préserver la surface des chemises et éviter une usure corrosive excessive. Ceci est généralement réalisé par réaction avec des sites basiques inclus dans le lubrifiant.

La capacité de neutralisation d'une huile peut être déterminée par son BN ou Base Number en anglais, caractérisant sa basicité. Il est habituellement mesuré suivant la norme ASTM D-2896 et est exprimé en équivalent en poids de potasse par gramme d'huile, ou mg de KOH/g. Le BN est un critère classique permettant d'ajuster la basicité des huiles à la teneur en soufre du fioul utilisé comme combustible moteur, afin de pouvoir neutraliser le soufre contenu dans ledit combustible et susceptible de se transformer en acide sulfurique.

Ainsi, plus la teneur en soufre d'un fioul est élevée, plus le BN d'une huile marine doit être élevé. C'est pourquoi on trouve sur le marché des huiles marines de BN variant, par exemple, de 5 à 100 mg KOH/g. Il y a donc nécessité d'ajuster le BN du lubrifiant à la teneur en soufre du combustible utilisé.

En outre, des préoccupations environnementales ont induit, dans certaines zones et notamment des zones côtières, des exigences plus importantes en matière de limitation du taux de soufre dans les fiouls utilisés sur les navires.

C'est le cas, par exemple, de la réglementation MARPOL Annexe 6 (Regulation for the Prevention of air pollution from ships) de l'IMO (International Maritime Organisation) qui est entrée en vigueur en mai 2005. Elle prévoit une teneur maximale en soufre de 4,5 % m/m des fiouls lourds (ceux qui alimentent les moteurs marins deux-temps), ainsi que la création de zones à émissions contrôlées en oxyde de soufre appelées SECAs (SOx Emission Control Areas). Les navires entrant dans ces zones devront utiliser des fiouls à teneur maximale en soufre de 1,5 % m/m, ou tout autre traitement alternatif visant à limiter les émissions en SOx pour respecter les valeurs spécifiées. La notation « % m/m » désigne le pourcentage massique d'un composé par rapport au poids total de la composition de fioul, ou de la composition lubrifiante, dans laquelle il est inclus.

Plus récemment le Comité MEPC (Marine Environment Protection Committee) s'est réuni en avril 2008 et a proposé des amendements à la réglementation MARPOL Annexe 6. Ces modifications sont résumées dans le tableau 1 ci-dessous. Elles présentent un scénario dans lequel les restrictions de teneur maximum en soufre deviennent plus sévères avec une teneur maximale mondiale limitée de 4,5% m/m à 3,5% m/m dès 2012. Les SECAs deviendront des ECAs (Emission Control Areas) avec une baisse complémentaire de la teneur maximum admissible en soufre de 1,5% m/m à 1,0% m/m dès 2010 et l'adjonction de nouvelles limites concernant les teneurs en NOx et les particules.

**Tableau 1 : Amendements à la réglementation MARPOL Annexe 6**

| | **Réglementation actuelle MARPOL Annexe 6** | |
|---|---|---|
| | | |

| | Limite générale | Limite pour les SECA's |
|---|---|---|
| **Teneur maximale en Soufre** | 4,50 % m/m | 1,50% m/m |
| | | |

| | **Amendements à MARPOL Annexe 6 (Réunion MEPC n°57 - avril 2008)** | |
|---|---|---|
| | | |

| | Limite générale | Limite pour les ECA's |
|---|---|---|
| **Teneur maximale en Soufre** | 3,50 % m/m au 1/01/2012 | 1,00 % m/m au 1/03/2010 |
| | 0,50 % m/m au 1/01/2020 | 0,10 % m/m au 1/01/2015 |

Les navires effectuant des liaisons trans-continentales utilisent d'ores et déjà plusieurs types de fiouls lourds en fonction des contraintes environnementales locales. Cette situation perdurera quel que soit le niveau final de la teneur maximale en soufre admissible dans les fiouls.

Ainsi pour la plupart des navires porte-containeurs actuellement en construction, il est prévu la mise en oeuvre de plusieurs bacs de combustible, pour un fioul « haute mer » à teneur en soufre élevée d'une part et pour un fioul « SECA » à teneur en soufre inférieure ou égale à 1,50 % m/m d'autre part.

Le basculement entre ces deux catégories de fiouls peut nécessiter l'adaptation des conditions d'opération du moteur, en particulier la mise en oeuvre de lubrifiants cylindre appropriés.

De ce fait, une première solution pour optimiser la lubrification cylindre d'un moteur deux-temps lent est alors la sélection du lubrifiant avec un BN adapté au fioul et aux conditions opératoires du moteur. Cette optimisation réduit la flexibilité d'opération du moteur et exige une technicité importante de l'équipage dans la définition des conditions dans lesquelles le basculement d'un type de lubrifiant sur un autre doit être réalisé. Une autre alternative consiste à disposer d'un lubrifiant unique et d'optimiser les conditions opératoires du moteur (taux de graissage ou autres paramètres de combustion) en fonction du combustible employé.

Cette optimisation pourrait être atteinte par une analyse en continu, ou en discontinu, du BN du lubrifiant afin de maintenir l'adéquation requise entre ledit BN dudit lubrifiant et la teneur en soufre du combustible alimentant le moteur ; l'objectif étant bien évidemment d'éviter le fonctionnement du moteur à BN trop faible qui est générateur de corrosions irréversibles des parties sensibles pistons-cylindres du moteur et une dégradation rapide des performances fonctionnelles du lubrifiant à l'intérieur du cylindre.

De plus, l'usure mécanique d'un moteur quatre temps ou deux temps (ou d'une machine) doit pouvoir être contrôlée et surtout maîtrisée, par l'analyse de certaines caractéristiques physico-chimiques ou intrinsèques de son lubrifiant. Par exemple, quand la mesure de la teneur spécifique de certains métaux (non limitativement Fe, Pb, Ni, Cr, Al, Cu, Sb, Ag) employés dans la constitution des parties sensibles du moteur (selon le design dudit moteur) révèle leur(s) présence(s) dans le lubrifiant analysé (sous forme métallique ou dissoute), la présence de ce(s) dits composé(s) peut signifier un phénomène d'arrachement dans les parties sensibles du moteur, aboutissant ainsi à une destruction partielle, voire importante du moteur. Il est donc important, voire primordial, de détecter le phénomène d'usure des parties métalliques du moteur au plus tôt, voire dès son origine. Cette détection doit donc passer par un suivi analytique rigoureux et constant du lubrifiant pendant le fonctionnement du moteur.

De la même façon que pour les métaux d'usure, il est important de contrôler la viscosité du lubrifiant, par exemple,
- afin de réduire au minimum la vitesse d'usure des parties sensibles, du moteur (ou de la machine), concernées par les contacts ou frottement, roulants ou glissants, objets de ladite lubrification,
- de limiter les échauffements,
- de réduire la consommation d'énergie, et
- de participer au refroidissement localisé.

Le contrôle de la viscosité du lubrifiant permet également de détecter rapidement la pollution dudit lubrifiant par un autre fluide, par exemples le combustible, le carburant, le liquide de refroidissement ou un autre lubrifiant.

De même, une teneur en eau excessive peut altérer rapidement les qualités fonctionnelles du lubrifiant, notamment par la formation de « boues » qui dégradent les qualités du lubrifiant et provoquent ainsi une augmentation de la vitesse de corrosion et, par conséquent, une augmentation de la vitesse d'usure des parties sensibles du moteur ou de la machine. La présence de bactéries dans l'eau est aussi une source importante de pollution qui peut conduire à la dégradation rapide dudit lubrifiant et aussi apporter une nuisance importante à la santé des opérateurs par contact avec ledit lubrifiant. La mesure en continu ou en discontinu de la viscosité, de la teneur en matériaux d'usure, de la teneur en eau offre donc une sérieuse opportunité pour le suivi du bon fonctionnement du moteur ou de la machine, pour l'amélioration des performances du moteur ou de la machine, l'amélioration de sa protection contre l'usure et contre la corrosion, afin in fine, d'améliorer sa durabilité.

Le lubrifiant circulant dans une machine ou un moteur contribue donc à assurer de nombreuses fonctions essentielles au fonctionnement d'un moteur ou d'une machine, mais il va subir des dégradations au cours dudit fonctionnement comme indiquées ci-dessus. Il convient donc de surveiller les caractéristiques du lubrifiant en service afin de prévenir les corrosions, les dégradations voire les avaries des machines et/ou moteurs.

Si le suivi analytique d'une huile lubrifiante en service et in fine, le suivi en continu, ou en discontinu, du fonctionnement d'un moteur, notamment marin, par l'analyse de son lubrifiant, est un puissant outil pour l'optimisation dudit fonctionnement du moteur ou de la machine et aussi pour prévenir les désordres mécaniques dudit équipement industriel, ce suivi avec les méthodes et les technologies disponibles dans l'Art pose néanmoins de nombreux problèmes.

En effet, il n'existe pas aujourd'hui de solutions techniques permettant à la fois d'assurer trois fonctions essentielles :
1/ restituer de nombreuses mesures (BN, viscosité, teneur en eau, teneur en particules, etc...),
2/ restituer ces mesures de façon continue pour la plupart, ou discontinue, avec des fréquences répétitives très rapides pour les autres (en quelques minutes ou dizaines de minutes) et surtout,
3/ effectuer ces mesures directement sur le lubrifiant en circulation dans le moteur ou la machine pour assurer une parfaite représentativité du couple échantillonnage et analyse.

Les solutions techniques disponibles dans l'Art requièrent toutes un prélèvement d'échantillon du lubrifiant en service, ce qui peut poser un problème évident de représentativité et aussi un problème d'évolution de cet échantillon dans le temps avant son analyse.

Les solutions actuelles sont en général dites, « Off line » , ou « At line » , ou encore « Mini laboratoire ».

Les analyses réalisées « Off Line », c'est-à-dire dans des laboratoires extérieurs, si elles s'avèrent satisfaisantes en termes de résultats, ne sont pas adaptées à la prise en compte de brusques variations de la qualité de l'huile, par exemple une variation rapide du BN ou une soudaine usure du moteur. En effet, la nécessité de transporter l'échantillon dans un laboratoire situé à plusieurs centaines de kilomètres du navire, implique que le résultat peut intervenir plusieurs jours, voire plusieurs semaines, voire encore plusieurs mois après la prise d'échantillon correspondante et donc devenir sans intérêt lors de sa réception parce que les conditions de fonctionnement du moteur ont été modifiées, ou tout simplement et à l'extrême, une dégradation mécanique, voire une casse moteur est survenue. L'optimisation en temps réel du moteur, ou de la machine, ou encore la détection précoce d'une dégradation ou d'une contamination du lubrifiant est donc rendue impossible par le délai trop important du retour des résultats. Il en est de même pour une optimisation en temps réel du fonctionnement d'un moteur marin comme par exemple le réglage de son taux de lubrification, en fonction du combustible et de sa teneur en soufre.

De plus, les délais importants dus aux transports des échantillons avant analyses peuvent être générateurs de phénomènes de dégénérescence de ces derniers, voire de l'apparition de microorganismes dans ces mêmes échantillons, par exemple au contact de l'air et de l'éventuelle humidité relative dans les flacons de transport des dits échantillons, pouvant provoquer un biais plus ou moins important sur les résultats de certains types d'analyses.

Des solutions ont été proposées pour remédier à ces différents inconvénients par des systèmes d'analyses dits « embarqués », lesdites analyses étant réalisées à partir d'un « mini-laboratoire » inclus dans une mallette transportable.

Si les analyses sont relativement simples à réaliser avec ces « mini laboratoires », en revanche les techniques d'analyses restent limitées, la périodicité des mesures est généralement de quelques jours à une semaine et surtout elles nécessitent une main d'oeuvre formée et qualifiée à bord du navire.

Il en est de même pour des analyses « At line », c'est-à-dire des analyses réalisées aussi à bord des navires mais à partir d'analyseurs spécifiques de type laboratoire. Ces analyses, qui représentent de facto, un investissement très important, une charge de travail supplémentaire pour le personnel de bord, ainsi qu'un temps opérateur non négligeable, ont encore des applications limitées et demandent des précautions importantes eu égard à leur spécificité analytique, notamment en termes d'étalonnages de l'analyseur, de son utilisation et de sa maintenance analytique. Ces analyses « At line » requièrent de plus une solide formation et beaucoup de compétence de la part des opérateurs.

C'est ainsi que EP1485696 décrit un procédé permettant de déterminer l'indice d'alcalinité totale (TBN) d'un lubrifiant de moteur marin en 3 étapes :
a) la mesure de la bande d'absorption infrarouge allant de 840 à 910 cm-1,
b) la détermination de la valeur de l'absorbance pour au moins un pic présentant un intérêt à l'intérieur de la dite fréquence de bande, et
c) le calcul du TBN du lubrifiant d'après la valeur de l'absorbance du pic à partir d'une ligne de base calculée.

Il en est de même dans WO 03/048763 qui décrit une méthode pour déterminer les contaminants dans un lubrifiant d'un moteur marin diesel. Cette méthode nécessite quatre actions de l'opérateur :
a) l'obtention d'un spectre UV pour un échantillon dit de référence aux propriétés connues et contenant des contaminants en quantités connues,
b) le développement d'un modèle de calibration,
c) les mesures sur les échantillons dans les mêmes conditions,
d) le calcul de la concentration des contaminants.

Comme indiqué ci-dessus, si des solutions existent pour suivre le fonctionnement d'un moteur marin par un suivi analytique de son lubrifiant, ces techniques ne permettent pas d'obtenir des résultats en continu, ou en discontinu, garants d'une traçabilité exhaustive dudit fonctionnement. De plus, elles demandent généralement une maîtrise et un savoir-faire de la part du marin opérateur et un temps dudit opérateur non négligeable pour un parfait suivi analytique.

Dans tous les cas des solutions évoquées ci-dessus, le suivi du couple soufre et BN et/ou la détection précoce d'une anomalie moteur par le suivi de la qualité de son lubrifiant ne sera pas systématiquement assurée et pourra par conséquent engendrer des périodes plus ou moins longues d'incertitudes pouvant être préjudiciables à l'environnement (trop de rejet de soufre dans l'atmosphère) ou au moteur, avec des risques de dommage(s), telle une casse irréversible car les symptômes précurseurs n'ont pas été détectés suffisamment tôt.

Il en est également de même avec la Demande Internationale PCT WO 03/091550 où les techniques utilisées sont les spectrométries de fluorescence de rayons X pour les mesures des teneurs en fer et en soufre respectivement sur le lubrifiant et le combustible et Infra-Rouge pour la mesure du BN du lubrifiant.

Il est à noter également que toutes ces solutions « At line » qui peuvent faire appel à des techniques spectroscopiques, par exemple rayons X, Infra-rouge ou encore Ultra-violet, nécessitent des appareillages sensibles et fragiles (trajets internes optiques) qui doivent faire l'objet de précautions quant à leur emplacement, manipulation, température, stockage, etc.

De plus, et de façon générale, ces différentes techniques analytiques ne sont pas particulièrement adaptées pour un fonctionnement en continu ou en discontinu avec des fréquences d'analyses rapides sur le lubrifiant.

En particulier, les solutions proposées sont coûteuses, nécessitant un matériel encombrant, un opérateur qualifié, requérant un laps de temps relativement long entre la prise d'échantillon et le résultat de l'analyse, et présentant des risques de perte d'échantillon ou de contamination de celui-ci et/ou ne pouvant pas être rapidement appliquées sur le système à étudier.

Le but de la présente invention vise à pallier en tout ou partie à ces inconvénients en proposant un dispositif et un procédé permettant d'obtenir directement sur le lubrifiant en service, des résultats d'analyses de façon continue ou discontinue, afin d'assurer la détection précoce d'une dégradation, d'une contamination du lubrifiant et permettre ainsi l'optimisation et/ou le contrôle du fonctionnement d'une machine ou d'un moteur, notamment thermique, et plus précisément marin, à partir du suivi de la qualité de son lubrifiant.

La présente invention peut permettre à la fois d'assurer trois fonctions essentielles :
1/ la restitution de nombreuses mesures (BN, viscosité, teneur en eau, teneur en particules, etc...),
2/ la restitution de ces mesures de façon continue pour la plupart ou discontinue avec des fréquences répétitives très rapides pour les autres (en quelques minutes ou dizaines de minutes) et
3/ la réalisation des mesures directement sur le lubrifiant en circulation dans le moteur ou la machine (en assurant ainsi la représentativité du couple échantillonnage et analyse).

De part son faible encombrement d'une part, et sa forte modularité d'autre part, le dispositif est facilement adaptable sur pratiquement tous les moteurs ou toutes les machines.

A cet effet, un premier objet de l'invention concerne un dispositif pour le suivi, en continu ou en discontinu, de l'évolution de la qualité d'un lubrifiant en circulation dans un équipement industriel (M), ledit dispositif comprenant, ou étant constitué essentiellement :
- d'un ensemble de distribution (2) du lubrifiant du type comprenant au moins un module de canalisation (6) dudit lubrifiant disposé sur le corps (5) dudit ensemble de distribution,
- au moins un composant fonctionnel (8) du type moyen de mesure pour la détermination d'au moins une caractéristique physico-chimique ou une caractéristique intrinsèque du lubrifiant connecté sur ledit module de canalisation, le composant fonctionnel étant un microcapteur ou un microanalyseur MEMs (Micro Electro Mechanical system- Micro Système ElectroMécanique) ou NEMs (Nano Electro Mechanical system- Nano Système ElectroMécanique), et ladite caractéristique étant choisie parmi la température, la pression, la viscosité, le BN (Base Number), la teneur en particules métalliques, la teneur en eau ;
- au moins un système de mesure (10) du signal généré par ledit composant fonctionnel , et
- au moins un système automatisé de contrôle (12) d'au moins un paramètre fonctionnel ou de composition, respectivement sur l'équipement industriel ou le lubrifiant.

Parmi les ensembles de distribution utilisables dans la présente invention, on peut citer celui décrit dans WO 2007/110504 A1. Celui-ci présente un encombrement réduit et comporte :
- un corps comprenant au moins une face plane sur laquelle est disposé au moins un module de canalisation du lubrifiant,
- un passage pour le lubrifiant ménagé à l'intérieur du corps,
- un filtre calibré situé à l'intérieur de l'ensemble de distribution pour filtrer le fluide lubrifiant avant son entrée dans le composant fonctionnel et éventuellement,
- un dispositif de préparation du lubrifiant en vue d'une analyse spécifique, connecté sur un module de canalisation additionnel, fixé sur le corps de l'ensemble de distribution.

Les dimensions de l'ensemble de distribution, support du composant fonctionnel peuvent aller de 100 mm à 300 mm pour sa longueur, et de 20 mm à 70 mm pour sa largeur, ainsi que sa hauteur. L'encombrement est donc limité et peut être inférieur ou égal à 300 mm x 70 mm x 70 mm.

Le module de canalisation est fixé sur la face plane du corps de l'ensemble de distribution conformément à la norme ANSI/ISA-76.00.02-2002 et plus récemment CEI 62339-1 :2006.

L'ensemble de distribution peut comprendre un dispositif de préparation de l'échantillon, en particulier disposé sur un module de canalisation. Cet ensemble de distribution peut être une unité de chauffage ou de refroidissement de l'échantillon, de réduction de la pression, de contrôle et/ou de maîtrise du débit, d'une filtration spécifique en vue de préparer l'échantillon pour une analyse particulière ou plusieurs analyses selon le(s) paramètre(s) à mesurer.

Au moins un composant fonctionnel est connecté sur un module de canalisation conformément aux recommandations NESSI (New Sampling and Sensor Initiative) pour les systèmes d'échantillonnages.

Dans l'invention, ce composant fonctionnel est un micro capteur ou un microanalyseur, encore appelé dans la technique MEMs (Micro Electro Mechanical system) ou NEMs (Nano Electro Mechanical system), permettant la mesure de la température et/ou de la pression du lubrifiant, de la viscosité, du BN, ou encore la quantité de particules de fer non dissoutes contenues dans le lubrifiant. Tout particulièrement, le microcapteur ou le microanalyseur est adapté à un module de canalisation.

Ces microanalyseurs sont commercialisés, par exemple, par les sociétés MEMs Schlumberger ou Vectron, notamment respectivement sous les noms commerciaux, Excalibur ou ViSmart™.

De façon plus générale, les caractéristiques physico-chimiques et/ou intrinsèques mesurées sur le lubrifiant peuvent être de différentes natures, par exemple :
- la mesure de la viscosité qui peut permettre de contrôler la capacité fonctionnelle du lubrifiant à assurer efficacement la protection des parties sensibles du moteur contre des échauffements anormaux localisés, lesdits échauffements pouvant conduire à un début de destruction du moteur, parfois rédhibitoire pour son fonctionnement,
- la mesure du BN qui peut permettre de contrôler la capacité du lubrifiant à protéger les parties sensibles du moteur contre les effets de la corrosion acide dus à la teneur en soufre du fioul de combustion,
- la mesure des teneurs en particules métalliques, en fer ou en autres métaux, présentes dans le lubrifiant, qui peut permettre de vérifier le bon fonctionnement du moteur (quand il y a des niveaux de teneurs très faibles des mesures) ou de détecter un début de désordre mécanique lié à l'arrachement de métal dans les parties mécaniques (quand les niveaux des teneurs mesurées augmentent significativement) qui peuvent être très sensibles et indispensables pour le bon fonctionnement du moteur, et
- la mesure de la teneur en eau, eau libre et/ou eau dissoute, qui peut permettre d'apprécier le bon fonctionnement en temps normal du moteur (ou de la machine) et des auxiliaires du circuit lubrifiant comme par exemple la filtration en continu du lubrifiant, la séparation eau huile assurée par le design de la caisse à huile et/ou, additionnellement, par une centrifugation.

Cette dernière mesure peut permettre notamment de détecter une cause accidentelle due à une entrée d'eau propre au moteur, ou propre au circuit réfrigérant du lubrifiant, ou encore due à un disfonctionnement des équipements auxiliaires de filtration, séparation et traitement du lubrifiant.

Généralement chaque microanalyseur est choisi pour la détermination d'une ou plusieurs caractéristique(s) bien déterminée(s). Il peut donc y avoir autant de microanalyseurs que de caractéristiques, ou ensemble de caractéristiques physico-chimiques ou intrinsèques à contrôler. En particulier, chaque caractéristique physico-chimique ou intrinsèque mesurée est associé un microanalyseur ou un micro capteur particulier.

Associé à ces microanalyseurs, un système électronique et/ou informatique, de mesure du ou des signaux générés par lesdits microanalyseurs, permet de mettre en forme et quantifier lesdits signaux, pour in fine, leur exploitation dans un système automatisé de commande d'au moins un paramètre fonctionnel et/ou de composition, respectivement sur l'équipement industriel et/ou du lubrifiant.

Ces résultats sont ensuite exploités pour agir sur, par exemple, le taux de graissage, la charge moteur, le fonctionnement d'un piston dans le cas d'un moteur deux-temps, le renouvellement (qui peut être partiel) de la charge huile d'un moteur quatre-temps ou tout paramètre de contrôle du fonctionnement du moteur ou de la machine.

Avantageusement l'ensemble de distribution du dispositif selon l'invention est disposé sur une canalisation située en aval du moteur représentative du produit sur lequel le contrôle doit être exercé.

Le lubrifiant peut être prélevé à partir d'un point sous pression du circuit de lubrification, par exemple plusieurs bars. Autrement dit, l'alimentation de l'ensemble de distribution en lubrifiant peut être un point sous pression de l'équipement industriel.

L'huile prélevée est introduite dans l'ensemble de distribution, analysée par au moins un microanalyseur, puis réinjectée dans ledit circuit de lubrification.

Encore plus avantageusement, deux dispositifs analogues peuvent être disposés en amont et en aval de l'équipement industriel.

En effet, un ensemble de distribution analogue peut être équipé de la même manière en filtre et composant(s) fonctionnel(s) et être disposé en amont du moteur ou de la machine. Ceci peut permettre de quantifier plus précisément, grâce à une analyse des résultats avant et après l'équipement industriel, par exemple un taux d'usure dudit équipement industriel, ou une surveillance du bon fonctionnement des circuits auxiliaires et de traitement du lubrifiant (refroidissement, filtration ou éventuelle pollution par de l'eau ou autre fluide).

Les faibles dimensions et l'importante modularité du dispositif et, plus précisément, de l'ensemble de distribution support du ou des modules de canalisation, lui-même ou eux-mêmes support(s) d'au moins un composant fonctionnel, peuvent permettre une localisation dudit ensemble de distribution in situ sur le moteur marin, c'est-à-dire au plus près de l'équipement industriel.

L'invention concerne également un procédé de contrôle d'un équipement industriel comprenant la circulation d'un lubrifiant, seul ou en mélange, ledit contrôle étant réalisé par le suivi, en continu ou en discontinu, d'au moins une caractéristique physico-chimique ou intrinsèque dudit lubrifiant en circulation, dans lequel
- le lubrifiant est introduit de manière continue ou discontinue dans le dispositif selon l'invention,
- au moins une caractéristique physico-chimique et/ou intrinsèque dudit lubrifiant est mesurée par un moyen d'analyse connecté sur un module de canalisation disposé sur le corps d'un ensemble de distribution, le moyen d'analyse étant un microcapteur ou un microanalyseur MEMs (Micro Electro Mechanical system-Micro Système ElectroMécanique) ou NEMs (Nano Electro Mechanical system- Nano Système ElectroMécanique), et
- la (ou les) valeur(s) mesurée(s) est(sont) traitée(s) par des moyens informatiques pour être ensuite restituée(s) sous forme de signaux adaptés afin de commander, en fonction de la nature et de la réponse de la caractéristique mesurée, des paramètres fonctionnels respectivement sur l'équipement industriel et/ou le lubrifiant.

Le suivi en continu d'au moins une caractéristique physico-chimique est une réponse en temps réel, sans interruption, de la grandeur mesurée par le microanalyseur. C'est le cas du suivi de la qualité de l'huile lubrifiante des moteurs marins quatre-temps semi-rapides à rapides ou deux-temps lents pour les huiles système.

Le suivi en discontinu d'au moins une caractéristique physico-chimique est une réponse cyclique de la grandeur mesurée par le microanalyseur. Par exemple, dans le cas du suivi de la qualité de l'huile cylindre, et plus précisément de l'huile égoutture, d'un moteur marin deux-temps lent pour lequel l'ensemble de distribution est d'abord empli par le lubrifiant, puis isolé du circuit dudit lubrifiant, pour permettre l'analyse par le micro capteur ou le microanalyseur. Un nouveau cycle recommence ensuite, espacé de quelques dizaines de secondes, voire de minutes ou tout simplement d'heures, suivant le type de mesure(s) à réaliser en conformité avec le(s) type(s) de phénomène(s) moteur ou machine à détecter ou à surveiller.

Le lubrifiant prélevé peut être réinjecté en continu après analyse dans le circuit de lubrification de l'équipement industriel.

Le suivi en continu d'au moins une caractéristique physico-chimique ou intrinsèque peut notamment être réalisé pour les huiles lubrifiantes des moteurs marins quatre-temps semi-rapides à rapides et les huiles systèmes des moteurs deux temps lents.

Le suivi en discontinu d'au moins une caractéristique physico-chimique ou intrinsèque peut être réalisé pour les huiles cylindres des moteurs marins deux temps lents.

Le paramètre fonctionnel de l'équipement industriel peut être la puissance du moteur, la nature du lubrifiant et/ou le taux de graissage.

Ce document concerne enfin l'utilisation du dispositif et du procédé objets de la présente invention à bord d'un navire pour le contrôle du fonctionnement d'un moteur, notamment d'un moteur quatre-temps semi-rapide à rapide ou d'un moteur deux temps lent.

Selon un autre de ses aspects, ce document a pour objet l'utilisation du dispositif ou du procédé selon l'invention pour permettre d'optimiser certains paramètres de fonctionnement, tel le taux de graissage du moteur, notamment dans le cas des moteurs deux-temps lents.

Selon encore un autre de ses aspects, ce document a pour objet l'utilisation du dispositif ou du procédé selon l'invention pour optimiser la valeur du BN du lubrifiant, en particulier en fonction du taux de soufre mesuré, par exemple en temps réel, sur le carburant, notamment le fioul.

Selon encore un autre de ses aspects, ce document a pour objet l'utilisation du dispositif ou du procédé selon l'invention pour optimiser la formulation du lubrifiant en continu, c'est-à-dire en temps réel et en fonction des résultats des mesures effectuées par les micro-capteurs, et/ou les microanalyseurs, et ainsi réduire la consommation de lubrifiant au niveau minimum requis pour le bon fonctionnement du moteur ou de l'équipement, et aussi améliorer l'impact environnemental par une réduction des émissions dans l'atmosphère. Cette optimisation peut notamment être réalisée en fonction du taux de soufre mesuré dans le fioul.

Bien entendu, cette variante peut être plus facilement réalisée dans la mesure où le navire dispose d'une unité de mélange (huile de base/additifs/booster ...).

L'invention peut être également, par exemple, utilisée pour le contrôle du fonctionnement d'une machine, par exemple tournante, ou tout simplement grâce à ses petites dimensions, au contrôle embarqué du fonctionnement d'un moteur automobile, voire de compétition pour son utilisation aux limites de ses performances mécaniques.

L'invention est maintenant décrite en référence au dessins annexés, non limitatifs, dans lesquels :

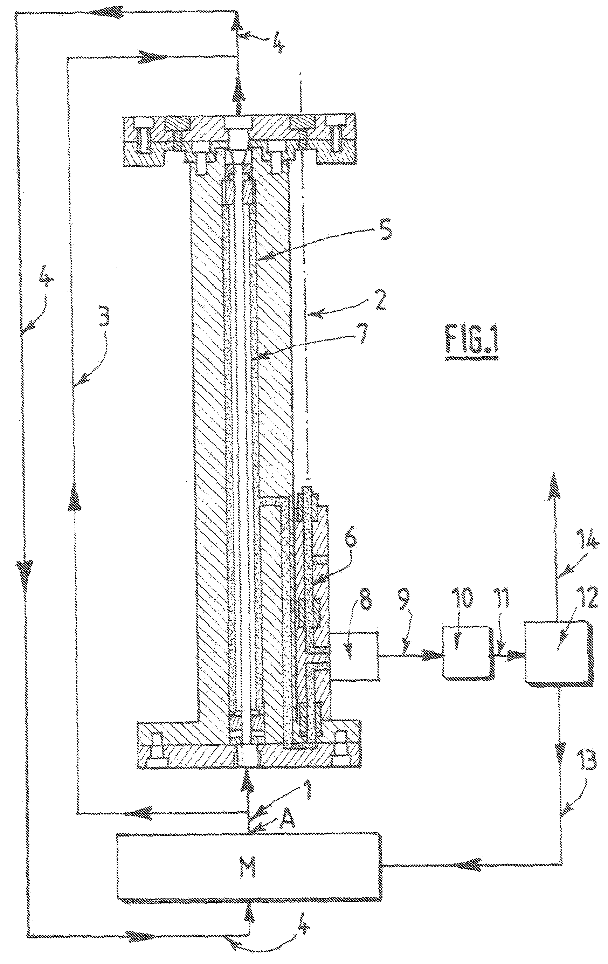

- la figure 1 est une représentation schématique du dispositif selon l'invention appliqué par exemple aux moteurs marins quatre-temps et aux huiles système des moteurs deux-temps lents ou à tout type de circuit de lubrification d'un équipement industriel fonctionnant avec un lubrifiant sous pression.

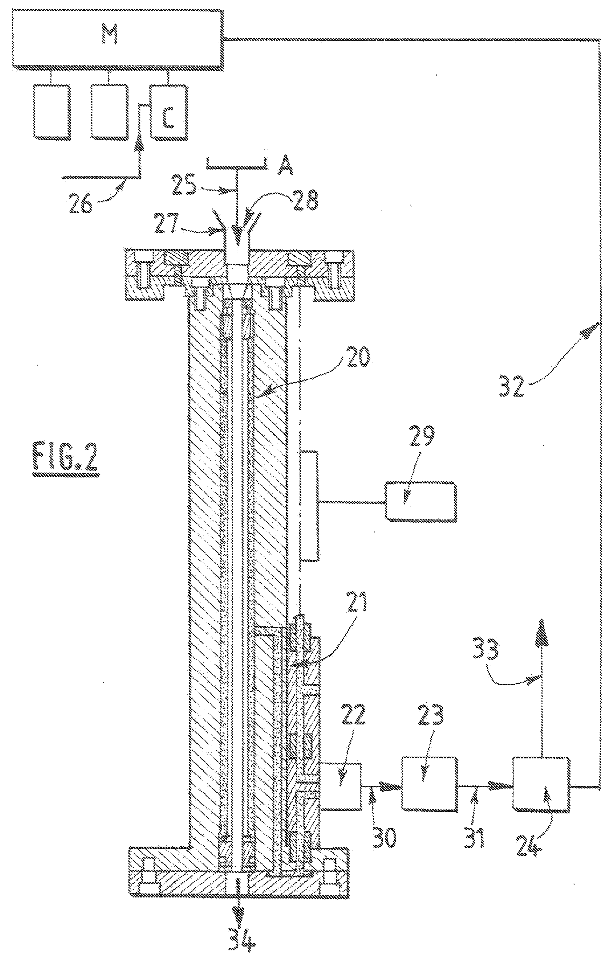

- La figure 2 est une représentation schématique du dispositif selon l'invention appliqué par exemple aux moteurs marins deux-temps, et plus précisément au suivi analytique des huiles cylindres de ces moteurs, lesdites huiles étant récupérées en bas desdits cylindre sous forme d'huiles égouttures. Plus généralement, il s'agit ici d'une analyse réalisée sur un fluide récupéré par égouttures discontinues à pression atmosphérique, égouttures dont le suivi analytique permet une optimisation.

Sur la figure 1 et pour les moteurs marins quatre-temps et deux-temps pour l'huile système, le dispositif comprend :
- un ensemble de distribution (2) disposé en aval d'un moteur marin (M),
- au moins un module de canalisation (6) disposé sur le corps de l'ensemble de distribution (2),
- au moins un composant fonctionnel (8) connecté sur le module de canalisation (6),
- au moins un système de mesure (10) du signal généré par le composant fonctionnel (8),
- au moins un système automatisé de contrôle (12) d'au moins un paramètre fonctionnel ou de composition, respectivement sur l'équipement industriel (ici le moteur marin) ou le lubrifiant en circulation.

Le lubrifiant est prélevé en continu à partir d'un point (A) du circuit de lubrification du moteur (M). Au point (A) la pression peut varier, par exemple, de 1 à 5 bars. Le point de prélèvement du lubrifiant est ici unique et situé en aval du moteur (M).

Le lubrifiant est introduit, par la canalisation (1), dans l'ensemble de distribution (2), ledit ensemble de distribution (2) étant by-passé par la canalisation (3), dans laquelle circule le lubrifiant, pour assurer le débit correct dans le circuit de lubrification du moteur (M).

En sortie de l'ensemble de distribution (2), le lubrifiant est réinjecté dans le circuit de lubrification (ici un circuit fermé) par la canalisation (4).

L'ensemble de distribution (2) est constitué d'un corps (5) sur lequel et plus précisément sur une de ses faces planes, est fixé un module de canalisation (6). Ce module de canalisation (6) permet l'alimentation en lubrifiant à analyser du composant fonctionnel (8). Un filtre (7) peut être présent, et notamment être disposé dans l'axe longitudinal de l'ensemble de distribution, ce filtre permettant d'assurer la filtration du lubrifiant avant son introduction dans le module de canalisation (6) et in fine dans le composant fonctionnel (8).

Bien évidemment, l'ensemble de distribution (2) peut être constitué de plusieurs faces planes sur lesquelles sont disposées plusieurs modules de canalisation (6), eux-mêmes connectés à plusieurs composants fonctionnels (8), encore appelés microcapteurs ou microanalyseurs ou MEMs ou NEMs. Cette dernière configuration permet, à partir d'un espace analyse très réduit, d'obtenir les réponses de plusieurs microanalyseurs, c'est-à-dire les réponses des mesures de plusieurs caractéristiques physico-chimiques ou intrinsèques déterminées en temps réel sur le lubrifiant.

Connecté au système de mesure (10) par un raccordement électrique (9), le composant fonctionnel (8) émet un signal représentatif de la caractéristique en cours de mesure qui sera mis en forme dans le dit système de mesure (10) pour commander ensuite le système automatisé (12) via la connexion électrique (11).

Suivant la nature de la caractéristique mesurée, suivant l'étalonnage du système automatisé (12), des informations sont envoyés pour le suivi et l'optimisation du moteur (M) via la liaison (13) ou, si le navire est équipé par exemple d'un ensemble automatisé d'appoint de lubrifiant neuf, des ordres de modifications de la composition du lubrifiant, sont envoyés via la liaison (14).

Sur la figure 2 et pour les moteurs marins deux-temps, notamment pour le suivi analytique des huiles cylindres et en particulier pour l'analyse des huiles égouttures, le dispositif suivant la présente invention est constitué de :
- un ensemble de distribution (20),
- au moins un module de canalisation (21) disposé sur le corps de l'ensemble de distribution (20),
- au moins un composant fonctionnel (22) connecté sur le module de canalisation (21),
- au moins un système de mesure (23) du signal généré par le composant fonctionnel (22),
- au moins un système automatisé de contrôle (24) d'au moins un paramètre fonctionnel ou de composition, respectivement sur l'équipement industriel ou le lubrifiant en circulation,
- au moins un dispositif (29) permettant d'exercer une pression sur le lubrifiant se trouvant à l'intérieur de l'ensemble de distribution.

Un seul cylindre (C) du moteur deux-temps (M) est représenté sur la figure 2, mais le dispositif décrit s'applique à chacun des cylindres du moteur marin.

Le cylindre (C) du moteur marin deux-temps (M) est alimenté en huile neuve par la canalisation (26) ; le système de graissage du cylindre étant du type à lubrifiant perdu, encore dénommé à « graissage perdu » dans la profession.

L'huile égoutture, contaminée par les produits de combustion, est prélevée au point bas (A) du cylindre, à la pression atmosphérique, et s'écoule en discontinu, via la canalisation (25) dans l'ensemble de distribution (20), par l'intermédiaire d'un collecteur (27).

Le débit d'écoulement dans le collecteur (27) peut varier par exemple de 0,1 à 30 % du taux de graissage initial.

Un dispositif de prétraitement de l'huile égoutture (28) est disposé dans le système de collecte (27) pour par exemple exercer une première filtration du lubrifiant avant son analyse.

Le cycle d'analyse est constitué des étapes suivantes :
a) la sortie au slop (34) est isolée afin de permettre le remplissage du dispositif d'analyse (20). Une purge en continu du dispositif d'analyse (20) peut être réalisée par l'arrivée du produit à analyser avant la fermeture de la sortie au slop (34),
b) quand l'ensemble du dispositif d'analyse (20) est rempli dans un minimum de temps grâce à la nécessité de faibles volumes utiles pour les micro capteurs ou microanalyseurs, un dispositif (29) isole automatiquement l'arrivée de l'huile égoutture,
c) comme pour les moteurs quatre-temps le composant fonctionnel (22) émet par la connexion électrique (30) un signal représentatif de la caractéristique en cours de mesure qui sera mis en forme dans le système de mesure (23) pour commander ensuite le système automatisé (24) via la connexion électrique (31).
c) en fin de cycle d'analyse, la sortie au slop (34) est ouverte afin de vidanger le dispositif d'analyse (20),
d) après cette vidange, le cycle d'analyse est réinitialisé avec la fermeture de la sortie au slop (34).

Suivant la nature de la caractéristique mesurée, suivant l'étalonnage du système automatisé (24), des informations pour le suivi et l'optimisation sont envoyées au moteur M via la liaison (32) ou, si le navire est équipé par exemple d'un ensemble automatisé du taux de graissage du moteur, des ordres de modification de ce taux de graissage via la liaison électrique (33).

Le dispositif et procédé, objets de la présente invention, permettent de réduire la consommation de lubrifiant au niveau minimum requis, de contrôler le bon fonctionnement du moteur afin d'exploiter celui-ci au niveau optimal, mais aussi de détecter efficacement une variation du BN, une variation de la viscosité, un début d'arrachement de métal, une arrivée d'eau accidentelle dans le circuit lubrifiant et ainsi de pouvoir réagir rapidement avant l'aggravation du phénomène pouvant conduire à la destruction plus ou moins partielle du moteur et l'immobilisation du dit moteur ou de l'équipement et in fine, l'immobilisation du navire en haute mer.

Les résultats des mesures en continu, ou en discontinu, des dits paramètres ci-dessus indiqués sont rendus disponibles à bord du navire très rapidement et notamment avec des délais nettement améliorés par rapport aux analyses conventionnelles de l'art.

En effet, ces mesures conventionnelles actuelles requièrent la prise d'échantillons, l'expédition de celui-ci dans un port, son transport vers un laboratoire en charge du type d'analyses requis, le retour des résultats analytiques à bord du navire, ce processus pouvant prendre plusieurs semaines pour détecter par exemple que la teneur en fer est limite ou la teneur en eau dans le lubrifiant est anormale, c'est-à-dire longtemps après l'apparition de l'anomalie, ladite anomalie pouvant être rédhibitoire pour le bon fonctionnement du moteur.

De plus, les mesures en ligne par des micro capteurs ou microanalyseurs offrent des performances en terme de répétabilité nettement supérieures à la reproductibilité des mesures d'un laboratoire, ladite reproductibilité étant fortement liée à la qualité et à la formation du personnel.

## Revendications

1. Dispositif pour le suivi, en continu ou en discontinu, de l'évolution de la qualité d'un lubrifiant en circulation dans un équipement industriel (M), ledit dispositif comprenant, ou étant constitué essentiellement :
- d'un ensemble de distribution (2) du lubrifiant du type comprenant au moins un module de canalisation (6) dudit lubrifiant disposé sur le corps (5) dudit ensemble de distribution (2),
- au moins un composant fonctionnel (8) du type moyen de mesure pour la détermination d'au moins une caractéristique physico-chimique ou une caractéristique intrinsèque du lubrifiant connecté sur ledit module de canalisation (6), le composant fonctionnel étant un microcapteur ou un microanalyseur MEMs (Micro Electro Mechanical system- Micro Système ElectroMécanique) ou NEMs (Nano Electro Mechanical system- Nano Système ElectroMécanique), et ladite caractéristique étant choisie parmi la température, la pression, la viscosité, le BN (Base Number), la teneur en particules métalliques, la teneur en eau ;
- au moins un système de mesure (10) du signal généré par ledit composant fonctionnel (8), et
- au moins un système automatisé de contrôle (12) d'au moins un paramètre fonctionnel ou de composition, respectivement sur l'équipement industriel (M) ou le lubrifiant.

2. Dispositif selon la revendication 1, **caractérisé en ce que** l'ensemble de distribution (2) comporte un filtre (7).

3. Dispositif selon l'une des revendications 1 ou 2, **caractérisé en ce que** l'ensemble de distribution (2) comporte un dispositif de préparation de l'échantillon.

4. Dispositif selon la revendications 3, **caractérisé en ce que** le dispositif de préparation de l'échantillon est disposé sur un module de canalisation (6).

5. Dispositif selon l'une quelconque des revendications 1 à 4, **caractérisé en ce que** le micro capteur ou le microanalyseur permet la mesure de la quantité de particules de fer non dissoutes contenues dans le lubrifiant.

6. Dispositif selon l'une des revendications 1 à 5, **caractérisé en ce que** à chaque caractéristique physico-chimique ou intrinsèque mesurée est associé un microanalyseur ou un micro capteur particulier.

7. Dispositif selon l'une quelconque des revendications 1 à 6, **caractérisé en ce que** l'ensemble de distribution est disposé sur une canalisation située en aval de l'équipement industriel (M).

8. Dispositif selon l'une quelconque des revendications 1 à 7, **caractérisé en ce que** l'alimentation de l'ensemble de distribution (2) en lubrifiant est un point (A) sous pression de l'équipement industriel (M).

9. Dispositif selon l'une quelconque des revendications 1 à 8, **caractérisé en ce que** deux dispositifs analogues sont disposés en amont et en aval de l'équipement industriel (M).

10. Procédé de contrôle d'un équipement industriel comprenant la circulation d'un lubrifiant, seul ou en mélange, ledit contrôle étant réalisé par le suivi, en continu ou en discontinu, d'au moins une caractéristique physico-chimique ou intrinsèque dudit lubrifiant en circulation, dans lequel ladite caractéristique est choisie parmi la température, la pression, la viscosité, le BN (Base Number), la teneur en particules métalliques, la teneur en eau, et
- le lubrifiant est introduit de manière continue ou discontinue dans le dispositif selon l'une quelconque des revendications 1 à 9,
- au moins une caractéristique physico-chimique et/ou intrinsèque dudit lubrifiant est mesurée par un moyen d'analyse connecté sur un module de canalisation disposé sur le corps d'un ensemble de distribution, le moyen d'analyse étant un microcapteur ou un microanalyseur MEMs (Micro Electro Mechanical system- Micro Système ElectroMécanique) ou NEMs (Nano Electro Mechanical system- Nano Système ElectroMécanique), et
- la (ou les) valeur(s) mesurée(s) est(sont) traitée(s) par des moyens informatiques pour être ensuite restituée(s) sous forme de signaux adaptés afin de commander, en fonction de la nature et de la réponse de la caractéristique mesurée, des paramètres fonctionnels respectivement sur l'équipement industriel et/ou le lubrifiant.

11. Procédé selon la revendication 10, **caractérisé en ce que** le lubrifiant prélevé est réinjecté en continu après analyse dans le circuit de lubrification de l'équipement industriel.

12. Procédé selon la revendication 10 ou 11, **caractérisé en ce que** le suivi en continu d'au moins une caractéristique physico-chimique ou intrinsèque est réalisé pour les huiles lubrifiantes des moteurs marins quatre-temps semi-rapides à rapides et les huiles systèmes des moteurs deux temps lents.

13. Procédé selon la revendication 10 ou 11, **caractérisé en ce que** le suivi en discontinu d'au moins une caractéristique physico-chimique ou intrinsèque est réalisé pour les huiles cylindres des moteurs marins deux temps lents.

14. Procédé selon l'une quelconque des revendications 10 à 13, **caractérisé en ce que** le paramètre fonctionnel de l'équipement industriel (M) est la puissance du moteur, la nature du lubrifiant, et/ou le taux de graissage.

## Patentansprüche

1. Vorrichtung zur kontinuierlichen oder nicht kontinuierlichen Nachverfolgung der Entwicklung der Qualität eines Schmiermittels, das in einer großtechnischen Einrichtung (M) zirkuliert, wobei die Vorrichtung im Wesentlichen Folgendes umfasst oder besteht aus:
- einer Verteilerbaugruppe (2) für das Schmiermittel vom Typ, der mindestens ein Leitungsmodul (6) des Schmiermittels umfasst, das an dem Körper (5) der Verteilerbaugruppe (2) angeordnet ist,
- mindestens einem Funktionsbauteil (8) vom Typ Messmittel zum Bestimmen von mindestens einer physikochemischen Eigenschaft oder einer intrinsischen Eigenschaft des Schmiermittels, das mit dem Leitungsmodul (6) verbunden ist, wobei das Funktionsbauteil ein Mikrosensor oder ein MEMS- (Micro Electro Mechanical System - mikroelektromechanisches System) oder NEMS- (Nano Electro Mechanical System - nanoelektromechanisches System) Mikroanalysator ist, und wobei die Eigenschaft aus der Temperatur, dem Druck, der Viskosität, der BN (Base Number), dem Metallpartikelgehalt, dem Wassergehalt ausgewählt wird;
- mindestens einem Messsystem (10) für das Signal, das von dem Funktionsbauteil (8) erzeugt wird, und
- mindestens einem automatisierten Kontrollsystem (12) für mindestens einen Funktions- oder Zusammensetzungsparameter, jeweils an der großtechnischen Einrichtung (M) oder dem Schmiermittel.

2. Vorrichtung nach Anspruch 1, **dadurch gekennzeichnet, dass** die Verteilerbaugruppe (2) ein Filter (7) umfasst.

3. Vorrichtung nach einem der Ansprüche 1 oder 2, **dadurch gekennzeichnet, dass** die Verteilerbaugruppe (2) eine Vorrichtung zur Vorbereitung der Probe umfasst.

4. Vorrichtung nach Anspruch 3, **dadurch gekennzeichnet, dass** die Vorrichtung zur Vorbereitung der Probe an einem Leitungsmodul (6) angeordnet ist.

5. Vorrichtung nach einem der Ansprüche 1 bis 4, **dadurch gekennzeichnet, dass** der Mikrosensor oder der Mikroanalysator die Messung der Menge der nicht gelösten Eisenpartikel ermöglicht, die in dem Schmiermittel enthalten sind.

6. Vorrichtung nach einem der Ansprüche 1 bis 5, **dadurch gekennzeichnet, dass** jeder gemessenen physikochemischen oder intrinsischen Eigenschaft ein eigener Mikroanalysator oder Mikrosensor zugeordnet ist.

7. Vorrichtung nach einem der Ansprüche 1 bis 6, **dadurch gekennzeichnet, dass** die Verteilerbaugruppe an einem Leitungssystem angeordnet ist, das der großtechnischen Einrichtung (M) nachgeschaltet ist.

8. Vorrichtung nach einem der Ansprüche 1 bis 7, **dadurch gekennzeichnet, dass** die Versorgung der Verteilerbaugruppe (2) mit Schmiermittel ein unter Druck stehender Punkt (A) der großtechnischen Einrichtung (M) ist.

9. Vorrichtung nach einem der Ansprüche 1 bis 8, **dadurch gekennzeichnet, dass** zwei analoge Vorrichtungen der großtechnischen Einrichtung (M) vorgeschaltet und nachgeschaltet angeordnet sind.

10. Verfahren zur Kontrolle einer großtechnischen Einrichtung, umfassend die Zirkulation eines Schmiermittels, allein oder in Mischung, wobei die Kontrolle durch die kontinuierliche oder nicht kontinuierliche Nachverfolgung von mindestens einer physikochemischen oder intrinsischen Eigenschaft des zirkulierenden Schmiermittels verwirklicht wird, wobei die Eigenschaft aus der Temperatur, dem Druck, der Viskosität, der BN (Base Number), dem Metallpartikelgehalt, dem Wassergehalt ausgewählt wird, und
- das Schmiermittel nach einem der Ansprüche 1 bis 9 kontinuierlich oder nicht kontinuierlich in die Vorrichtung eingebracht wird,
- mindestens eine physikochemische und/oder intrinsische Eigenschaft des Schmiermittels durch ein Analysemittel gemessen wird, das an ein Leitungsmodul angeschlossen ist, das am Körper einer Verteilerbaugruppe angeordnet ist, wobei das Analysemittel ein Mikrosensor oder ein MEMS- (Micro Electro Mechanical System - mikroelektromechanisches System) oder NEMS- (Nano Electro Mechanical System - nanoelektromechanisches System) Mikroanalysator ist, und
- der (oder die) gemessene(n) Wert(e) mit Informatikmitteln verarbeitet wird (werden), um anschließend in Form von Signalen wiederhergestellt zu werden, die geeignet sind, in Abhängigkeit von der Art und dem Verhalten der gemessenen Eigenschaft Funktionsparameter jeweils an der großtechnischen Vorrichtung und/oder dem Schmiermittel zu steuern.

11. Verfahren nach Anspruch 10, **dadurch gekennzeichnet, dass** das entnommene Schmiermittel nach der Analyse kontinuierlich wieder in den Schmiermittelkreislauf der großtechnischen Einrichtung eingespritzt wird.

12. Verfahren nach Anspruch 10 oder 11, **dadurch gekennzeichnet, dass** die kontinuierliche Nachverfolgung von mindestens einer physikochemischen oder intrinsischen Eigenschaft für die Schmieröle von mittelschnell bis schnell laufenden Viertakt-Schiffsmotoren und die Systemöle von langsam laufenden Zweitaktmotoren verwirklicht wird.

13. Verfahren nach Anspruch 10 oder 11, **dadurch gekennzeichnet, dass** die kontinuierliche Nachverfolgung von mindestens einer physikochemischen oder intrinsischen Eigenschaft für die Zylinderöle von langsam laufenden Zweitakt-Schiffsmotoren verwirklicht wird.

14. Verfahren nach einem der Ansprüche 10 bis 13, **dadurch gekennzeichnet, dass** der Funktionsparameter der großtechnischen Einrichtung (M) die Motorleistung, die Art des Schmiermittels und/oder der Schmierungsgrad ist.

## Claims

1. A device for continuously or discontinuously monitoring the change of quality of a lubricant circulating through an industrial apparatus (M), said device comprising, or essentially consisting of:
- a lubricant distribution assembly (2) of the type comprising at least one lubricant piping module (6) positioned on the body (5) of said distribution assembly (2),
- at least one functional component (8) of the measurement means type for determining at least one physicochemical property or one intrinsic property of the lubricant connected to said piping module (6), the functional component being a microsensor or a microanalyzer MEMs (Micro Electro Mechanical system) or NEMs (Nano Electro Mechanical system), and said property being chosen from the temperature, the pressure, the viscosity, the BN (Base Number), the metallic particles content, the water content;
- at least one measurement system (10) for measuring the signal generated by said functional component (8), and
- at least one automated control system (12) for checking at least one functional parameter or composition parameter of the industrial apparatus (M) or of the lubricant, respectively.

2. The device as claimed in claim 1, **characterized in that** the distribution assembly (2) comprises a filter (7).

3. The device as claimed in claim 1 or 2, **characterized in that** the distribution assembly (2) comprises a sample preparation device.

4. The device as claimed in claim 3, **characterized in that** the sample preparation device is positioned on a piping module (6).

5. The device as claimed in any one of claims 1 to 4, **characterized in that** the microsensor or the microanalyzer allows the measurement of the quantity of undissolved iron particles contained in the lubricant.

6. The device as claimed in one of claims 1 to 5, **characterized in that** a specific microanalyzer or a specific microsensor is associated with each physicochemical or intrinsic property measured.

7. The device as claimed in any one of claims 1 to 6, **characterized in that** the distribution assembly is positioned on piping situated downstream of the industrial apparatus (M).

8. The device as claimed in any one of claims 1 to 7, **characterized in that** the lubricant feed to the distribution assembly (2) is a pressurized point (A) of the industrial apparatus (M).

9. The device as claimed in any one of claims 1 to 8, **characterized in that** two similar devices are positioned upsteam and downstream of the industrial apparatus (M).

10. A method for checking an industrial apparatus involving the circulation of a lubricant, alone or in a mixture, said check being performed by continuously or discontinuously monitoring at least one physicochemical or intrinsic property of said circulating lubricant, in which method said property is chosen from the temperature, the pressure, the viscosity, the BN, the metallic particle content, the water content, and
- the lubricant is introduced continuously or discontinuously into the device as claimed in any one of claims 1 to 9,
- at least one physicochemical and/or intrinsic property of said lubricant is measured by an analysis means connected to a piping module positioned on the body of a distribution assembly, the analysis means being a microsensor or a microanalyzer MEMs (Micro Electro Mechanical system) or NEMs (Nano Electro Mechanical system), and
- the measured value(s) is/are processed by computerized means so that they can then be returned in the form of signals suited to controlling, according to the nature and response of the measured property, functional parameters of the industrial apparatus and/or the lubricant, respectively.

11. The method as claimed in claim 10, **characterized in that** the lubricant withdrawn is continuously reinjected after analysis into the lubricating circuit of the industrial apparatus.

12. The method as claimed in claim 10 or 11, **characterized in that** the continuous monitoring of at least one physicochemical or intrinsic property is performed on the lubricating oils of medium-speed to high-speed four-stroke marine engines and on the system oils of low-speed two-stroke engines.

13. The method as claimed in claim 10 or 11, **characterized in that** the discontinuous monitoring of at least one physicochemical or intrinsic property is performed on the cylinder oils of low-speed two-stroke marine engines.

14. The method as claimed in any one of claims 10 to 13, **characterized in that** the functional parameter of the industrial apparatus (M) is the power of the engine, the type of lubricant and/or the level of lubrication.
